Europäisches Patentamt

European Patent Office

Office européen des brevets

(11) Veröffentlichungsnummer: **0 582 755 A1**

# EUROPÄISCHE PATENTANMELDUNG

(21) Anmeldenummer: **92250200.0**

(51) Int. Cl.⁵: **G06F 15/401**

(22) Anmeldetag: **02.08.92**

(43) Veröffentlichungstag der Anmeldung:
**16.02.94 Patentblatt 94/07**

(84) Benannte Vertragsstaaten:
**AT CH DE FR GB IT LI SE**

(71) Anmelder: **SOFT GENE, ENTWICKLGS. U. VERTRIEBSGES. F. MOLEKULARBIOLOGISCHE SOFTWARE mbH Offenbacher Strasse 5 D-14197 Berlin(DE)**

(72) Erfinder: **The designation of the inventor has not yet been filed**

(74) Vertreter: **Erich, Dieter Patentanwalt August-Bebel-Ring 36 D-15751 Niederlehme bei Berlin (DE)**

(54) **Verfahren zum Einordnen von Informationsträgern in Datenbanken.**

(57) Die Erfindung betrifft ein Verfahren zum Einordnen von Informationen in Datenbanken für die Durchführung von DNA- und Proteinsequenzvergleichen.

Der Erfindung liegt die Aufgabe zugrunde, die Nachteile bisher bekannter Verfahren zur datenbankgerechten Aufbereitung von DNA- und Proteinsequenzen für deren Vergleich zu beseitigen. Diese Nachteile, die im Rechenzeitaufwand oder mangelnder Signifikanz der beim Vergleich ermittelten Treffer zu sehen sind, werden erfindungsgemäß dadurch vermieden, daß die Sequenzen in Strukturwörter zerlegt werden, die nicht nur die Abfolge der Nukleotide oder Aminosäuren beinhalten, sondern auch Informationen über deren Struktur und über Ähnlichkeitsmerkmale.

Entsprechend dem erfindungsmäßen Verfahren werden unter Berücksichtigung der Tatsache, daß die Nukleotide in der DNA und die Aminosäuren in den Proteinen nicht zufällig verteilt sind, für den DNA-Sequenzvergleich das Strukturwort NucIndex und für den Proteinsequenzvergleich die Strukturwörter IdenIndex und ChemIndex gebildet. Durch die Hinzuahme der Strukturparameter Strukturwortlänge und Gewichtung (SigS) zu den Strukturwörtern und das Zulassen von Mismatches an definierten Stellen von NucIndex und ChemIndex ermöglicht das offenbarte Verfahren einen schnellen Sequenzvergleich bei gleichzeitig hoher statistischer Signifikanz der Ergebnisse.

EP 0 582 755 A1

Die Erfindung betrifft ein Verfahren zum- Einordnen von Informationsträgern in Datenbanken für die Durchführung von DNA- und Proteinsequenzvergleichen, bei denen die zerlegten Datenketten mit ihren Eigenschaften mit denen von Sequenzen verglichen werden.

Es ist bekannt daß der genetische Bauplan, das Genom, z.B. beim Menschen durch ca. 3 Milliarden Buchstaben eines Alphabets aus nur vier verschiedenen Buchstaben festgelegt ist. Die vier Buchstaben repräsentieren die Bausteine aus den vier Nukleotiden mit den Basen Adenin, Thymin, Guanin und Cytosin (abgekürzt A, G, C und T) der Desoxyribonukleinsäure (engl. abgekürzt DNA). Der Bauplan in Form der DNA enthält ca. 120000 Sätze, die Gene genannt werden. In den Genen ist der Aufbau der biochemischen Produktions- Transport- und Werkzeugmaschinen und einiger Baumaterialien (zusammen Genprodukte genannt) beschrieben. Diese Genprodukte sind Proteine, deren Bausteine aus zwanzig Aminosäuren bestehen. Eine Übersetzung dieser Beschreibung gelang mehr als zwanzig Jahren durch die Aufklärung des "Genetischen Codes".

Um Erhaltung, Reaktion, Gestalt und Fortpflanzung eines Organismus zugewährleisten, muß die DNA jedoch außerdem Informationen darüber enthalten, welches und wieviel vom jeweiligen Genprodukt wann hergestellt werden muß. Dieser Vorgang wird Expression der Gene genannt, er ist qualitativ (welche?) quantitativ (wieviel?) und zeitlich (wann?) gesteuert. Die koordinierte Steuerung einer Vielzahl von Genen im Rahmen eines Expressionsprogramms wird wahrscheinlich durch die spezifische "Verpackung" der Gene als Chromatin im Zellkern erreicht.

Die Bearbeitung all dieser Fragestellungen erfordert die Analyse von DNA-und Protein-Sequenzen (Analyse der jeweiligen Bausteinfolge) Die neuen, experimentell ermittelten Sequenzen müssen dann mit bereits vorhandenen Sequenzen verglichen werden, um Ähnlichkeiten oder Identität feststellen zu können. Daraus können Rückschlüsse auf den evolutionären Verwandtschaftsgrad zwischen Spezies gezogen werden. Ein weiteres meist sehr viel wichtigeres Ergebnis solcher Sequenzvergleiche sind Erkenntnisse über die regulatorische Funktion von DNA-Sequenzen und die biochemische Funktion von Proteinen. Bisher bekannte DNA-Sequenzen sind in Europa im wesentlichen in der "EMBL Nukleotide Sequence Database" und in den USA in der Datenbank "GenBank" niedergelegt. Protein-Sequenzen werden ebenfalls zentral in Europa in der "SWISS-PROT" Protein Sequence Database" oder dem MIPS Projekt gespeichert. In den USA gilt als repräsentative Protein-Sequenz Datenbank die unter dem Namen PIR (Protein Identification Research) gepflegte Bank des NBFR (National Biomodical Research Foundation). Alle diese Banken gleichen ihren Datenbestand miteinander ab, d.h. enthalten zu jede Zeitpunkt zu ca. 90 % die gleichen Sequenzen. Auch die Formate sind im wesentlichen homologiert. Zum augenblicklichen Zeitpunkt sind ca. 60.000 DNA-Sequenzen mit ca. 80 x $10^6$ Aminosäuren gespeichert. Die durchschnittliche Länge der DNA-Sequenzen liegt bei 1300 Nukleotiden, die der Protein-Sequenzen bei 400 Aminosäuren. Die Datenbanken wachsen zur Zeit pro Jahr um ca. 40 %.

Herkömmliche Computer aber auch solche mit paralleler Architektur vergleichen lange Buchstabenfolgen (Strings) nur sehr langsam. Die Rechenzeit ist proportional zum Produkt aus den Anzahlen der Bausteine der beiden zu vergleichenden Sequenzen. Dies bedeutet in der Praxis, daß der Vergleich einer neuen DNA-Sequenz mit allen in den oben erwähnten Datenbanken zur Zeit bekannten DNA-Sequenzen auf Großrechnern Stunden auf kleineren Workstations Tage dauert.

Die Fragestellung ist allerdings ungleich schwieriger, wenn nicht nur festgestellt werden soll, ob die neue Sequenz mit einer aus dem Datenbestand identisch ist, sondern wenn ähnliche Sequenzen gefunden werden sollen. Durch den biologischen Mechanismus der Evolution wurden im Laufe der Phylogenese Veränderungen hervorgerufen, welche die genetische Information des jeweiligen Organismus an die Erfordernisse seiner Umwelt angepaßt haben. Diese Veränderungen, die auch jederzeit während der Ontogenese (Vermehren von Organismen durch einfache Teilung oder sexuelle Fortpflanzung) entstehen, werden als Mutationen bezeichnet. Für das Problem des Sequenzvergleichs äußern sich Mutationen durch nicht identische Bausteine innerhalb ansonsten identischer Abschnitte (Mismatches) oder durch Lücken in einer der Sequenzen im Vergleich zur anderen (Gaps). Da unter Umständen Sequenzen, die biologisch homolog sind (d.h. gleiche Funktion haben) derart mutiert sind, daß sie nur noch in 20 bis 30 % ihrer Bausteine übereinstimmen, müssen Datenbanken und Suchverfahren mit diesem Phänomen effektiv umgehen können. Mit den zur Zeit am höchsten entwickelten Verfahren kann die Suche nach Sequenzen, die zu einer bestimmten Sequenz (Query Sequenz) ähnlich sind, in ca. 30 Minuten CPU-Zeit auf Großrechnern durchgeführt werden. In der Praxis sind ein bis zwei Stunden bis zum Erhalt der Ergebnisse realistische Zeiten. Dies ist für die Erfordernisse großer Sequenzierungsprojekte, die pro Tag unter Umständen die Reihenfolge von 50000 Nukleotiden in ca. 100 einzelnen Sequenzen ermitteln, um mindestens eine Größenordnung zu langsam. Allein die erste grobe Suche nach Ähnlichkeiten zu Sequenzen im zur Zeit bekannten Datenbestand würde 3000 CPU-Minuten (100 bis 200 Stunden effektive Ergebniszeit) dauern.

Die eingefügten Protokolle Nummer 1, Nummer 2 und Nummer 3 entsprechende Seiten 3.1 3.2, 3.3, belegen die Richtigkeit der Feststellungen und zeigen die Mängel des Standes der Technik, die für den mit der Materie vertrauten Fachmann darin zu erkennen sind, daß die Ermittlung der dargestellten Datenreihen einen hohen Rechenaufwand erfordert. Damit ist es nicht möglich, in vertretbaren Zeitverläufen abrufbare Angaben zu erhalten.

Eine um Größenordnungen schnellere Lösung ist die Zerlegung der Strings in Sequenzwörter, die in einem alphabetischen Katalog gespeichert werden. Nach diesem Verfahren dauert der Vergleich einer 500 Nukleotide langen Query Sequenz mit allen Sequenzen einer entsprechenden Sequenwörter-Datenbank ein bis zwei Minuten. Die Vergleichszeit ist nur linear abhängig von der Länge der Query Sequenz und wird in der Praxis nur durch die Geschwindigkeit des

## Alignment for MYC$HUMAN vs MYC2$XENLA

```
Current comparison; is Identity compared
X-Sequence: MYC$HUMAN(439 aa);
Analyzed from: 1 to: 439
Y-Sequence: MYC2$XENLA(420 aa);
Analyzed from: 1 to: 420
Minimal window size: 3; Number of mismatches: 0
100 % compared; 79 matches found;
X-Sequence aligned from: 1 to: 437
Y-Sequence aligned from: 1 to: 419
Gap penalty: 3, Mismatch penalty: 1

Number of gaps inserted in MYC$HUMAN: 21
Number of gaps inserted in MYC2$XENLA: 39
Number of mismatches remaining in alignments: 214
Number of identities: 183
Similarity: 39.79%

••• DrawLegend
Fre, 31. Jul 1992   16:58 Uhr
Upper sequence is MYC$HUMAN
Lower sequence is MYC2$XENLA


      §   ::::::    :   2:: :::::::      3::    :
    1 MPLNVSFTNR NYDLDYDSVQ PYFYCDEEEN FYQQQQQSEL QPPAPSEDIW KKFELLPTPP    60
      S||M??|??? M|M?M|M?:| |?|???M||| |M::?M|M?M |||||||||| ||||||||||
    1 MPLNANFPSK NYDYDYDL_Q PCFFFLEEEN FY__HQQSRL QPPAPSEDIW KKFELLPTPP    57


        ::: :::::::::: :::::::::: :::::::::: :::::::::: ::::::::::
   61 LSPSRRSGLC SPSYVAVTPF SLRGDNDGGG GSFSTADQLE MVTELLGGDM VNQSFICDPD   120
      ||||||M??? |????????? ???????||? ?????????? ????||???? ??????????
   58 LSPSRRSSQS SLFPSTADQL EMVTEFLGGD MVNQSFICEA DDEALLKSIV IQDCMWSGFS   117


      :::::::::: :::::::::: :::::::::: :::::::::: :::::::::: ::::::::::
  121 DETFIKNIII QDCMWSGFSA AAKLVSEKLA SYQAARKDSG SPNPARGHSV CSTSSLYLQD   180
      ?????????? ????????|? ?????|???? ??|??????? ||???????? ??|???????
  118 AAAKLEKVVS EKLASYQASR KESALSTSQC QSQPPQSPLK SPSCDGSLNL GGTNRSSHEF   177


      ::::::::::             ::::   10:::::::::: :::::::::: ::::::::::
  181 LSAAASECID PSVVFPYPLN DS____SSPK SCASQDSSAF SPSSDSLLSS TESSPQGSPE   236
      |????|?|?M |||||||||| |M::::M|M? ?????????? |??|?|??|? ?|???????|
  178 LQDPSSDCVD PSVVFPYPLN DSISNASSPC QDLMLETPPI SSNSSSSESE DEQEDDDDDE   237


      :::::::::: :::::::::: :::::::::: :::::::::: :::::::::: :::::   10
  237 PLVLHEETPP _T____TSS DSEEEQEDEE EIDVV_S_V EKRQA_PGKR SES_GSPSAG   285
      ?????||??? :|:::::|?| ???|?????? ?????:|::| ?||??:|??? ???:?M|M??
  238 DCDEEEEIDV VTVEKRQTAS RRMESGSHSQ SSRPHHSPLV LKRCHVPIHQ HNYAASPSTK   297


      +::::::::: ::::::    : :::::::::: :::::::::: :::::::::: :::::
  286 GH__S_KPPH __SP_LVLKR CHVSTHCHNY AAPPSTRKDY PAAKRVKLDS VRVLRQISNN   339
      ??::||:|??? ::|?:?M|M: :::::::::: :::::::::: :::::::::: :::::M||||
  298 VDYVSSKRAK LESNVRVLK_ _____ _____ _____ _____QISNN   321


        :         :   :                  :          :::::
  340 RKCTSPRSSD TEENVKRRTH NVLERQFRNE LKRSFFALRD QIPELENNEK APKVVILKKA   399
      ||M?M||||M ?M|M?M|||| |||||||||| |M?M||||| M?|???M||| ||||||||||
  322 RKCASPRSSD SEENDKRRTH NVLERQFRNE LKLSFFALRD QVPRWRNNEK APKVVILKKA   381


        :::::::::: :::::::::: :::::    : :::    §
  400 TAYILSVQAE EQKLISEEDL LRKRREQLKH KLEQLRNSCA                        439
      M?|??|?|?? |??||?|??? |??|?M|M? ?|?M|||M
  382 TEYAISMQED ERRLIRETEQ LKYRKEQLKQ RLQQLRNSV                         420
```

Seite 3.2

**Alignment for MYC$HUMAN vs MYC$RAT**                          page 1

```
Current comparison; is Identity compared
X-Sequence: MYC$HUMAN(439 aa);
Analyzed from: 1 to: 439
Y-Sequence: MYC$RAT(439 aa);
Analyzed from: 1 to: 439
Minimal window size: 3; Number of mismatches: 0
100 % compared; 88 matches found;
X-Sequence aligned from: 1 to: 437
Y-Sequence aligned from: 1 to: 437
Gap penalty: 3, Mismatch penalty: 1

Number of gaps inserted in MYC$HUMAN: 2
Number of gaps inserted in MYC$RAT: 2
Number of mismatches remaining in alignments: 34
Number of identities: 401
Similarity:  90.94%

••• DrawLegend
Fre, 31. Jul 1992   16:56 Uhr
Upper sequence is MYC$HUMAN
Lower sequence is MYC$RAT


        $         :                  :          :      :
    1 MPLNVSFTNR NYDLDYDSVQ PYFYCDEEEN FY_QQQQQSE LQPPAPSEDI WKKFELLPTP   59
      S|||||M?M| |||||||||| ||M?M||||| |M:M||M:<| |||||||||| ||||||||||
    1 MPLNVSFANR NYDLDYDSVQ PYFICDEEEN FYHQQQQ_SE LQPPAPSEDI WKKFELLPTP   59


                       ::  :::::::::      :              :
   60 PLSPSRRSGL CSPSYVAV_T PFSLRGDNDG GGGSFSTADQ LEMVTELLGG DMVNQSFICD   118
      |||||||||| |||||||M:| ?||?|?|?M| ||M?M||||| ||M?M||||| ||||||||||
   60 PLSPSRRSGL CSPSYVAVAT SFSPREDDDG GGGNFSTADQ LEMMTELLGG DMVNQSFICD   119


                                                 ::::
  119 PDDETFIKNI IIQDCMWSGF SAAAKLVSEK LASYQAARKD SGSPNPARGH SVCSTSSLYL   178
      |||||||||| |||||||||| |||||||||| |||||||||| M?|??M|||| ||||||||||
  120 PDDETFIKNI IIQDCMWSGF SAAAKLVSEK LASYQAARKD STSLSPARGH SVCSTSSLYL   179


         :                          :::::::   :         :      :::
  179 QDLSAAASEC IDPSVVFPYP LNDSSSPKSC ASQDSSAFSP SSDSLLSSTE SSPQGSPEPL   238
      ||M?M||||| |||||||||| ||||||||||M ?|?||?M|M? M||||||M:M ||M???M|||
  180 QDLTAAASEC IDPSVVFPYP LNDSSSPKSC TSSDSTAFSS SSDSLLSS_E SSPRATPEPL   238


                       :              :::       ::::
  239 VLHEETPPTT SSDSEEEQED EEEIDVVSVE KRQAPGKRSE SGSPSAGGHS KPPHSPLVLK   298
      |||||||||| |||||||M?M |||||||||| ||M?|?M||| ||M????M|| ||||||||||
  239 VLHEETPPTT SSDSEEEQDD EEEIDVVSVE KRQPPAKRSE SGSSPSRGHS KPPHSPLVLK   298


                       :         :       :          :      :
  299 RCHVSTHQHN YAAPPSTRKD YPAAKRVKLD SVRVLRQISN NRKCTSPRSS DTEENVKRRT   358
      |||||||||| |||||||||| |||||M?M|| M?M|M?M||| |||M?M|||| |||||M?M|||
  299 RCHVSTHQHN YAAPPSTRKD YPAAKRAKLD SGRVLKQISN NRKCSSPRSS DTEENDKRRT   358


                                                 :::      :
  359 HNVLERQRRN ELKRSFFALR DQIPELENNE KAPKVVILKK ATAYILSVQA EEQKLISEED   418
      |||||||||| |||||||||| |||||||||| |||||||||| ||||||||M ?|?M|||M?M
  359 HNVLERQRRN ELKRSFFALR DQIPELENNE KAPKVVILKK ATAYILSVQA DEHKLISEKD   418


                  $
  419 LLRKRREQLK HKLEQLRNSC A                                            439
      |||||||||| |||||||M |
  419 LLRKRREQLK HKLEQLRNSG A                                            439
```

5

## Alignment for MYC$HUMAN vs MYCL$MOUSE

```
Quick comparison; is Identity compared
X-Sequence: MYC$HUMAN(439 aa);
Analyzed from: 1 to: 439
Y-Sequence: MYCL$MOUSE(368 aa);
Analyzed from: 1 to: 368
Minimal window size: 3; Number of mismatches: 0
8 matches found;
X-Sequence aligned from: 15 to: 435
Y-Sequence aligned from: 12 to: 352
Gap penalty: 3, Mismatch penalty: 1

Number of gaps inserted in MYC$HUMAN: 1
Number of gaps inserted in MYCL$MOUSE: 81
Number of mismatches remaining in alignments: 251
Number of identities: 89
Similarity: 19.85%

.... DrawLegend
Fre, 31. Jul 1992    17:00 Uhr
Upper sequence is MYC$HUMAN
Lower sequence is MYCL$MOUSE


                      §  10+ ::::::::::: :::::::::: ::::::::::: :::::::::::
      1 MPLNVSFTNR NYDLDYDSVQ PYFYCDEEEN FYQQQQQSEL QPPAPSEDIW KKFELLPTPP    60
        s           |  M|M??? ?????????? ?????????? ?||??????? ??????|???
      1    MDFDSYQ HYFYDYDCGE DFYRSTAPSE DIWKKFELVP SPPTSPPWGS GPGAVDPASG    57


        ::::::::::: :::::::::: ::::::::::: :::::::::: ::::::::::: :::::::::::
     61 LSPSRRSGLC SPSYVAVTPF _SLRGDNDGG GGSFSTADQL EMVTELLGGD MVNQSFICDP   119
        ??|??????? ?????|???? :|???????? ?????????? ?????????? ?|????????
     58 INPGEPWPGG GAGDEAESRG HSKAWGRNYA SIIRRDCMWS GFSARERLER VVSDRLAPGA   117


        ::::::::::: :::::::::: ::::::::::: :::::::::  ::::::::: :::::::::::
    120 DDETFIKNII IQDCMWSGFS AAAKLVSEKL ASYQAARKDS GSPNPARGHS VCSTSSLYLQ   179
        ??????|??? ??|???|??? ?????????? ?????????M |M??|????? ??????????
    118 PRGNPPKAPA TPDGTPSLEA SNPAPATQCQ LGEPKTQACS GSESPSDSEG EEIDVVTVEK   177


        ::::::::::: :::::::::: ::::::::::: :::::::::: ::::::::::: :::::::::::
    180 DLSAAASECI DPSVVFPYPL NDSSSPKSCA SQDSSAFSPS SDSLLSSTES SPQGSPEPLV   239
        ??|??????? ???|?????? ?????????? ?|???????? ??????|?|? ?|???|????
    178 RRSLDIRKPV TITVRADPLD PCMKHFHISI HQQQHNYAAR FPPESCSQEG DPEPGPQEEA   237


        ::::::::::: :::    :::      10+:::: ::::::::::: ::::::::::: :::::::::::
    240 LHEETPPTTS SDSEEEQEDE EEIDVVSVEK RQAPGKRSES GSPSAGGHSK PPHSPLVLKR   299
        ???|?|???? ???M|M:|:M ||M:|:|??? ?:::|::||? ::|?::::|? ::|:|:::|?
    238 PEIEAPKEKE EEEEEE_E_E EEI_V_SPPP V___G__SEA __PQ____SC __H_P___KP   276


        ::::::::::: :::::::::: ::::::::::: :::::::::: ::::::::::: :::::  10
    300 CHVSTHQHNY AAPPSTRKDY PAAKRVKLDS VRVLRQISNN RKCTSPRSSD TEENVKRRTH   359
        ::||??:::: :::::|?:|? ?::||:!??? ?::|::::::: ::::::::::: :|::?M|M::
    277 __VSSD____ _____TE_DV T__KR_YNHN F__L_____ _____ E__RKRR__   298


        +:::::::::: :::::::          :::::::::         10+ ::::::::::: :::::::::::
    360 NVLERQRRNE LKRSFFALRD QIPELENNEK APKVVILKKA TAYILSVQAE EQKLISEEDL   419
        |?|:|?|::: ::::|?M||| M?|?|????M ||||||M?|| ??|:|::||: :::|???|??
    299 NDL_RSR___ ____FLALRD QVPTLASCSK APKVVILSKA LEY_L__QA_ ___LVGAEKK   343


        ::::::::::: :::  §
    420 LRKRREQLKH KLEQLRNSCA                                             439
        ???::|::|: ::?M|M
    344 MAT__E__K_ __RQLRCRQQ QLQKRIAYLS GY                              368
```

Beispiel 1.0

Einstellungen für NucIndex

┌─────────────────────────────────────────────────────────────────┐
│ ▤▢▤▤▤▤ **Parameters for Sequence Similarity Search** ▤▤▤▤▤ │
│                                                                   │
│  ┌──────────────────────────────────────────────────────────┐   │
│  │  [ **Query Sequence** ]  MYC C Exon 2                     │   │
│  │                                                           │   │
│  │              from:│1    │   to:│1534 │                    │   │
│  │                   └──────┘       └──────┘                  │   │
│  │                     1..1523        12..1534               │   │
│  └──────────────────────────────────────────────────────────┘   │
│  ┌─Database:─────────────────────────────────────────────────┐  │
│  │  ◉ **Nucleic Acid Database**                               │  │
│  │  ○ **Protein Database**                                    │  │
│  └────────────────────────────────────────────────────────────┘  │
│  ┌─Subset of Sequences:───────────────────────────────────────┐  │
│  │  ☒ **Primates**      ☐ **Viruses**       ☐ **Prokaryotes** │  │
│  │  ☒ **Rodents**       ☒ **Invertebrates** ☐ **Phages**      │  │
│  │  ☒ **Mammals**       ☒ **Plants**        ☐ **Organelles**  │  │
│  │  ☒ **Other Vertebrates** ☒ **Fungi**     ☐ **Synthetic**   │  │
│  └────────────────────────────────────────────────────────────┘  │
│    ☒ **Show SigA Distribution**      [ **Start Similarity Search** ] │
└─────────────────────────────────────────────────────────────────┘

Beispiel 1.1

Verteilung der Strukturwort-Treffer

Beispiel 1.2

**Selected Results of Sequence Similarity Search**

Query Sequence: MYC C Exon 2
compared from: 1 to: 1534
with: Nucleic Acids Database, Release 29
subsets: PRI ROD MAM VRT INV PLN FUN
date: 30 JUL 92
Minimum SigA: 2
Total Matches: 19638
Matches/Entry: 467.6 ±

| No. | Identifier | Length | SigA | SigB | Short Description | page 1 |
|-----|-----------|--------|------|------|-------------------|--------|
| 1 | HSMYCC | 8082 | 1523 | 1443 | Human c-myc oncogene | |
| 2 | HSMYCE12 | 3324 | 1399 | 1328 | Human myc-oncogene exon 1 and exon 2 | |
| 3 | HSMYCG2 | 3605 | 1395 | 1323 | Human (gh) germline c-myc proto-oncogene, 5' flank throu | |
| 4 | HSMYCLYA | 1936 | 1096 | 1030 | Human translocated c-myc oncogene (Ly65) in mu switch re | |
| 5 | HSMYCBL3 | 1037 | 1033 | 973 | Human (b122) translocated t(8;14) c-myc oncogene, exon 2 | |
| 6 | HSMYCF2 | 951 | 948 | 889 | Human fetal liver c-myc proto-oncogene, exon 2 and flank | |
| 7 | HSCMYC02 | 961 | 923 | 864 | Human aberrant c-myc exon 2 from LY67 Burkitt lymphoma c | |
| 8 | HSMYC2 | 3922 | 808 | 735 | Human translocated c-myc gene in Raji Burkitt lymphoma c | |
| 9 | HSMYCPOB | 2041 | 778 | 711 | Human c-myc-P64 mRNA, initiating from promoter P0, (HLmy | |
| 10 | HSMYC1 | 2121 | 777 | 710 | Human mRNA encoding the c-myc oncogene. | |
| 11 | HSMYCDT | 2154 | 773 | 706 | Human (Daudi) translocated t(8;14) c-myc oncogene mRNA, | |
| 12 | HSON01 | 773 | 636 | 579 | Human translocated c-myc gene transcription initiation s | |
| 13 | HSMYCPOA | 1023 | 523 | 463 | Human c-myc-P64 mRNA, initiating from promoter P0, (HLmy | |
| 14 | FSMYCCA2 | 900 | 451 | 393 | Cat c-myc gene, complete cds. | |
| 15 | FCFTT | 2721 | 451 | 382 | Cat feline proviral (FTT) v-myc gene, complete cds. | |
| 16 | MAHCRMYC | 1978 | 387 | 321 | Woodchuck mRNA for fused hcr and myc genes | |
| 17 | MMB3 | 1733 | 332 | 267 | Mouse t(15;12) translocation region: c-myc exon 1 (chrom | |
| 18 | MMCMYC1 | 2300 | 306 | 238 | Mouse normal c-myc gene and translocated homologue from | |
| 19 | HSCC1S14 | 34379 | 288 | 193 | Human cosmid clone HDAB (1S149) insert DNA, complete. | |
| 20 | HSADAG | 36741 | 273 | 178 | Human adenosine deaminase gene, complete cds. | |
| 21 | HSHBB | 73326 | 273 | 171 | Human beta globin region on chromosome 11. | |
| 22 | HSCSF1PO | 35100 | 261 | 166 | Human c-fms proto-oncogene for CSF-1 receptor | |
| 23 | HSBMYH7 | 28438 | 244 | 151 | Homo sapiens beta-myosin heavy chain (MYH7) gene, comple | |
| 24 | HSTPA | 36594 | 235 | 140 | Human tissue plasminogen activator (t-PA) gene, complete | |
| 25 | HSGHCSA | 66495 | 240 | 139 | Human growth hormone (GH-1 and GH-2) and chorionic somat | |
| 26 | HSHPRTB | 56736 | 234 | 134 | Human hypoxanthine phosphoribosyltransferase (HPRT) gene | |
| 27 | HSIGLAMB | 33737 | 228 | 133 | Human lambda-immunoglobulin constant region complex (ger | |
| 28 | GGMYC | 4984 | 204 | 129 | Chicken cellular myc proto-oncogene, complete cds. | |
| 29 | HSCBMYHC | 25000 | 218 | 126 | Human gene for cardiac beta myosin heavy chain | |
| 30 | HSATP1A2 | 26668 | 209 | 117 | Human Na,K-ATPase subunit alpha 2 (ATP1A2) gene, complet | |
| 31 | MMBGCXD | 55856 | 213 | 113 | Mouse beta-globin complex DNA for y, bh0, bh1, b1 and b2 | |
| 32 | GDCOL6A2G | 27430 | 205 | 112 | Chicken Col6A2 gene for type VI collagen subunit alpha2 | |
| 33 | HSIGCMUDE | 19795 | 201 | 112 | Human immunoglobulin C(mu) and C(delta) heavy chain gene | |
| 34 | HSRYR | 15346 | 194 | 107 | Human ryanodine receptor mRNA, complete cds. | |
| 35 | HSG6PDGEN | 20114 | 190 | 101 | Human complete G6PD gene for glucose-6-phosphate dehydro | |
| 36 | GGCMYCA | 3517 | 170 | 98 | Chicken tumor 10 c-myc DNA, exons 2 and 3. | |
| 37 | HSHKATPC | 17201 | 183 | 95 | Human gastric H,K-ATPase catalytic subunit gene, complet | |
| 38 | HSLDLRRL | 14896 | 180 | 94 | Human mRNA for LDL-receptor related protein | |
| 39 | HSATPGG | 15115 | 169 | 82 | Human gastric (H+ + K+)-ATPase gene, complete cds. | |
| 40 | HSC4AA2 | 13048 | 163 | 78 | Human complement component C4A gene, exons 10 through 41 | |
| 41 | HSFIXG | 38059 | 161 | 65 | Human factor IX gene, complete cds. | |
| 42 | CEUNC22 | 47081 | 163 | 65 | Caenorhabditis elegans unc-22 gene for twitchin. | |

Beispiel 1.3

**Selected Results of Sequence Similarity Search**

Query Sequence: MYC C Exon 2
compared from: 1 to: 1534
with: Nucleic Acids Database, Release 29
subsets: PRI ROD MAM VRT INV PLN FUN
date: 30 JUL 92
Minimum SigA: 2
Total Matches: 19638
Matches/Entry: 467.6 ±

| No. | Identifier | Length | SigA | SigB | Short Description | page 1 |
|---|---|---|---|---|---|---|
| 1 | HSON01 | 773 | 636 | 579 | Human translocated c-myc gene transcription initiation s | |
| 2 | FSMYCCA2 | 900 | 451 | 393 | Cat c-myc gene, complete cds. | |
| 3 | HSMYCF2 | 951 | 948 | 889 | Human fetal liver c-myc proto-oncogene, exon 2 and flank | |
| 4 | HSCMYC02 | 961 | 923 | 864 | Human aberrant c-myc exon 2 from LY67 Burkitt lymphoma c | |
| 5 | HSMYCPOA | 1023 | 523 | 463 | Human c-myc-P64 mRNA, initiating from promoter P0, (HLmy | |
| 6 | HSMYCBL3 | 1037 | 1033 | 973 | Human (b122) translocated t(8;14) c-myc oncogene, exon 2 | |
| 7 | MMB3 | 1733 | 332 | 267 | Mouse t(15;12) translocation region: c-myc exon 1 (chrom | |
| 8 | HSMYCLYA | 1936 | 1096 | 1030 | Human translocated c-myc oncogene (Ly65) in mu switch re | |
| 9 | MAHCRMYC | 1978 | 387 | 321 | Woodchuck mRNA for fused hcr and myc genes | |
| 10 | HSMYCPOB | 2041 | 778 | 711 | Human c-myc-P64 mRNA, initiating from promoter P0, (HLmy | |
| 11 | HSMYC1 | 2121 | 777 | 710 | Human mRNA encoding the c-myc oncogene. | |
| 12 | HSMYCDT | 2154 | 773 | 706 | Human (Daudi) translocated t(8;14) c-myc oncogene mRNA, | |
| 13 | MMCMYC1 | 2300 | 306 | 238 | Mouse normal c-myc gene and translocated homologue from | |
| 14 | FCFTT | 2721 | 451 | 382 | Cat feline proviral (FTT) v-myc gene, complete cds. | |
| 15 | HSMYCE12 | 3324 | 1399 | 1328 | Human myc-oncogene exon 1 and exon 2 | |
| 16 | GGCMYCA | 3517 | 170 | 98 | Chicken tumor 10 c-myc DNA, exons 2 and 3. | |
| 17 | HSMYCG2 | 3605 | 1395 | 1323 | Human (gh) germline c-myc proto-oncogene, 5' flank throu | |
| 18 | HSMYC2 | 3922 | 808 | 735 | Human translocated c-myc gene in Raji Burkitt lymphoma c | |
| 19 | GGMYC | 4984 | 204 | 129 | Chicken cellular myc proto-oncogene, complete cds. | |
| 20 | HSMYCC | 8082 | 1523 | 1443 | Human c-myc oncogene | |
| 21 | HSC4AA2 | 13048 | 163 | 78 | Human complement component C4A gene, exons 10 through 41 | |
| 22 | HSLDLRRL | 14896 | 180 | 94 | Human mRNA for LDL-receptor related protein | |
| 23 | HSATPGG | 15115 | 169 | 82 | Human gastric (H+ + K+)-ATPase gene, complete cds. | |
| 24 | HSRYR | 15346 | 194 | 107 | Human ryanodine receptor mRNA, complete cds. | |
| 25 | HSHKATPC | 17201 | 183 | 95 | Human gastric H,K-ATPase catalytic subunit gene, complet | |
| 26 | HSIGCMUDE | 19795 | 201 | 112 | Human immunoglobulin C(mu) and C(delta) heavy chain gene | |
| 27 | HSG6PDGEN | 20114 | 190 | 101 | Human complete G6PD gene for glucose-6-phosphate dehydro | |
| 28 | HSCBMYHC | 25000 | 218 | 126 | Human gene for cardiac beta myosin heavy chain | |
| 29 | HSATP1A2 | 26668 | 209 | 117 | Human Na,K-ATPase subunit alpha 2 (ATP1A2) gene, complet | |
| 30 | GDCOL6A2G | 27430 | 205 | 112 | Chicken Col6A2 gene for type VI collagen subunit alpha2 | |
| 31 | HSBMYH7 | 28438 | 244 | 151 | Homo sapiens beta-myosin heavy chain (MYH7) gene, comple | |
| 32 | HSIGLAMB | 33737 | 228 | 133 | Human lambda-immunoglobulin constant region complex (ger | |
| 33 | HSCC1S14 | 34379 | 288 | 193 | Human cosmid clone HDAB (1S149) insert DNA, complete. | |
| 34 | HSCSF1PO | 35100 | 261 | 166 | Human c-fms proto-oncogene for CSF-1 receptor | |
| 35 | HSTPA | 36594 | 235 | 140 | Human tissue plasminogen activator (t-PA) gene, complete | |
| 36 | HSADAG | 36741 | 273 | 178 | Human adenosine deaminase gene, complete cds. | |
| 37 | HSFIXG | 38059 | 161 | 65 | Human factor IX gene, complete cds. | |
| 38 | CEUNC22 | 47081 | 163 | 65 | Caenorhabditis elegans unc-22 gene for twitchin. | |
| 39 | MMBGCXD | 55856 | 213 | 113 | Mouse beta-globin complex DNA for y, bh0, bh1, b1 and b2 | |
| 40 | HSHPRTB | 56736 | 234 | 134 | Human hypoxanthine phosphoribosyltransferase (HPRT) gene | |
| 41 | HSGHCSA | 66495 | 240 | 139 | Human growth hormone (GH-1 and GH-2) and chorionic somat | |

10

Beispiel 1.4

Keine Ähnlichkeit

Plot of MYC C Exon 2 vs.

Sequence Comparison

X-Axis:
MYC C Exon 2
from: 1   to:  1534

Y-Axis: HSCSF1PO

from: 1   to: 35100

Beispiel 1.5

Geringe Ähnlichkeit

Plot of MYC C Exon 2 vs. GGCMYCA

Sequence Comparison

X-Axis:
MYC C Exon 2
from: 1  to: 1534

Y-Axis:
GGCMYCA
from: 1  to: 3517

Beispiel 1.6

Ähnlichkeit

**Plot of MYC C Exon 2 vs. MMCMYC1**

Sequence Comparison

X-Axis:
MYC C Exon 2
from: 1 to: 1534

Y-Axis:
MMCMYC1
from: 1 to: 2300

Beispiel 1.7

Große Ähnlichkeit

**Plot of MYC C Exon 2 vs. FSMYCCA2**

Sequence Comparison

X-Axis:
MYC C Exon 2
from: 1  to: 1534

Y-Axis:
FSMYCCA2
from: 1  to: 900

Beispiel 1.8

Identität

## Plot of MYC C Exon 2 vs. HSMYCC

Sequence Comparison

X-Axis:
MYC C Exon 2
from: 1  to: 1534

Y-Axis:
HSMYCC
from: 1  to: 8082

Beispiel 2.0

Einstellungen für ProIden

**Parameters for Sequence Similarity Search**

[Query Sequence] MYC$HUMAN

        from: 1    to: 439

        1..436      4..439

┌Database:
○ Nucleic Acid Database
◉ Protein Database

┌Mode of Comparison:
◉ Identical Amino Acids
○ Chemical Similarity

☒ Show SigA Distribution     [Start Similarity Search]

Beispiel 2.1

Verteilung der Strukturwort-Treffer

**Distribution of SigA**

SigA
400
300
200
100
0

1          10          100          1000          10000

Number of Results

32 results selected of 15367

**Minimal SigA:**

28

5 .. 436

Cancel

Load Results

Beispiel 2.2

Selected Results of Sequence Similarity Search

Query Sequence: MYC$HUMAN
 compared from: 1 to: 439
           with: Protein Database, Release 20
           mode: identity in for subsequent amino acids
           date: 31 JUL 92
 Minimum SigA: 161
 Total Matches: 4885
 Matches/Entry: 152.7 ±

| No. | Identifier | Length | SigA | SigB | Short Description                                                        page 1 |
|-----|-----------|--------|------|------|--------------------------------------------------------------------------------|
| 1   | MYC$HUMAN | 439    | 436  | 382  | MYC PROTO-ONCOGENE PROTEIN (GENE NAME: C-MYC) - HOMO ENS                        |
| 2   | MYC$PANTR | 439    | 428  | 374  | MYC PROTO-ONCOGENE PROTEIN (GENE NAME: C-MYC) - PAN LODY                        |
| 3   | MYC$FELCA | 439    | 343  | 289  | MYC PROTO-ONCOGENE PROTEIN (GENE NAME: C-MYC) - FELIS S                         |
| 4   | MYC$FLV   | 439    | 340  | 286  | MYC TRANSFORMING PROTEIN (GENE NAME: V-MYC) - FELINE EMI                        |
| 5   | MYC$FLVTT | 437    | 332  | 278  | MYC TRANSFORMING PROTEIN (GENE NAME: V-MYC) - FELINE EMI                        |
| 6   | MYC$MOUSE | 439    | 327  | 273  | MYC PROTO-ONCOGENE PROTEIN (GENE NAME: C-MYC) - MUS ULUS                        |
| 7   | MYC1$MARMO| 465    | 327  | 273  | TRANSFORMING PROTEIN HCR-MYC - MARMOTA MONAX DCHUCK)                            |
| 8   | MYC$RAT   | 439    | 323  | 269  | MYC PROTO-ONCOGENE PROTEIN (GENE NAME: C-MYC) - RATTUS E                        |
| 9   | MYC$CHICK | 416    | 182  | 129  | MYC PROTO-ONCOGENE PROTEIN (GENE NAME: C-MYC) - GALLUS U                        |
| 10  | MYC$AVIM2 | 421    | 182  | 129  | MYC TRANSFORMING PROTEIN (GENE NAME: V-MYC) - AVIAN OCYT                        |
| 11  | MYC$AVIOK | 416    | 174  | 121  | MYC TRANSFORMING PROTEIN (GENE NAME: V-MYC) - AVIAN OVIR                        |
| 12  | MYC$AVIMC | 422    | 173  | 120  | MYC TRANSFORMING PROTEIN (GENE NAME: V-MYC) - AVIAN OCYT                        |
| 13  | MYC$AVIMD | 422    | 165  | 112  | MYC TRANSFORMING PROTEIN (GENE NAME: V-MYC) - AVIAN OCYT                        |
| 14  | MYC2$XENLA| 420    | 150  | 97   | MYC II PROTO-ONCOGENE PROTEIN (GENE NAME: C-MYC II) - PU                        |
| 15  | MYC$AVIME | 423    | 144  | 91   | MYC TRANSFORMING PROTEIN (GENE NAME: V-MYC) - AVIAN OVIR                        |
| 16  | MYC1$XENLA| 419    | 143  | 90   | MYC I PROTO-ONCOGENE PROTEIN (GENE NAME: C-MYC I) - PUS                         |
| 17  | MYC$SALGA | 414    | 118  | 65   | MYC PROTO-ONCOGENE PROTEIN (GENE NAME: C-MYC) GMENT) - S                        |
| 18  | MYCN$CHICK| 441    | 60   | 6    | N-MYC PROTO-ONCOGENE PROTEIN (GENE NAME: N-MYC) - US GAL                        |
| 19  | MYCN$HUMAN| 464    | 53   | -1   | N-MYC PROTO-ONCOGENE PROTEIN (GENE NAME: N-MYC) - HOMO E                        |
| 20  | MYCN$MOUSE| 462    | 50   | -4   | N-MYC PROTO-ONCOGENE PROTEIN (GENE NAME: N-MYC) - MUS UL                        |
| 21  | MYCB$RAT  | 178    | 40   | -5   | B-MYC TRANSFORMING PROTEIN (GENE NAME: B-MYC) GMENT) - R                        |
| 22  | MYC2$MARMO| 454    | 46   | -8   | N-MYC 2 PROTO-ONCOGENE PROTEIN (GENE NAME: N-MYC2) - OTA                        |
| 23  | MYCL$HUMAN| 364    | 39   | -13  | L-MYC PROTO-ONCOGENE PROTEIN (GENE NAME: MYCL1 OR C) - H                        |
| 24  | MYCL$MOUSE| 368    | 37   | -15  | L-MYC PROTO-ONCOGENE PROTEIN (GENE NAME: L-MYC) - MUS UL                        |
| 25  | RYNR$HUMAN| 5032   | 43   | -35  | RYANODINE RECEPTOR (GENE NAME: RYR1 OR RYDR) - HOMO ENS                         |
| 26  | HMCU$DROME| 2175   | 34   | -36  | HOMEOBOX PROTEIN CUT (GENE NAME: CT) - DROSOPHILA NOGAST                        |
| 27  | TEGU$EBV  | 3149   | 35   | -38  | LARGE TEGUMENT PROTEIN (GENE NAME: BPLF1) - EIN-BARR VIR                        |
| 28  | RYNR$RABIT| 5037   | 39   | -39  | RYANODINE RECEPTOR - ORYCTOLAGUS CUNICULUS (RABBIT)                             |
| 29  | PRDI$HUMAN| 2717   | 32   | -40  | DNA-BINDING PROTEIN PRDII-BF1 - HOMO SAPIENS (HUMAN)                            |
| 30  | TRX$DROME | 3759   | 34   | -41  | TRITHORAX PROTEIN (GENE NAME: TRX) - DROSOPHILA NOGASTER                        |
| 31  | TEGU$HSV11| 3164   | 28   | -45  | LARGE TEGUMENT PROTEIN (VIRION PROTEIN UL36) (GENE : UL3                        |
| 32  | ACVA$PENCH| 3746   | 28   | -47  | L-(ALPHA-AMINOADIPYL)-L-CYSTEINYL-D-VALINE SYNTHETASE 6.                        |

Beispiel 2.3

Keine Ähnlichkeit

Plot of MYC$HUMAN vs. HMCU$DROME

Sequence Comparison

X-Axis:
MYC$HUMAN
from: 1  to: 439

Y-Axis:
HMCU$DROME
from: 1  to: 2175

Beispiel 2.4

Geringe Ähnlichkeit

Plot of MYC$HUMAN vs. MYCL$MOUSE

Sequence Comparison

X-Axis:
MYC$HUMAN
from: 1  to: 439

Y-Axis:
MYCL$MOUSE
from: 1  to: 368

Beispiel 2.5

Ähnlichkeit

Plot of MYC$HUMAN vs. MYC2$XENLA

Sequence Comparison

X-Axis:
MYC$HUMAN
from: 1  to: 439

Y-Axis:
MYC2$XENLA
from: 1  to: 420

21

Beispiel 2.6

Große Ähnlichkeit

Plot of MYC$HUMAN vs. MYC$RAT

Sequence Comparison

X-Axis:
MYC$HUMAN
from: 1   to: 439

Y-Axis:
MYC$RAT
from: 1   to: 439

Beispiel 2.7

Identität

Beispiel 3.0

Einstellungen für ProChemIndex

```
┌─────────────────────────────────────────────────────────────┐
│ ▤□▤▤▤▤▤  Parameters for Sequence Similarity Search ▤▤▤▤ │
├─────────────────────────────────────────────────────────────┤
│  ┌─────────────────────────────────────────────────────────┐ │
│  │ │ Query Sequence │ MYC$HUMAN                            │ │
│  │                                                          │ │
│  │                  from: │1    │   to: │439 │             │ │
│  │                        1..436         11..439           │ │
│  └─────────────────────────────────────────────────────────┘ │
│  ┌─Database:───────────────────────────────────────────────┐ │
│  │                                                          │ │
│  │  ○ Nucleic Acid Database                                 │ │
│  │  ◉ Protein Database                                      │ │
│  └─────────────────────────────────────────────────────────┘ │
│  ┌─Mode of Comparison:─────────────────────────────────────┐ │
│  │                                                          │ │
│  │  ○ Identical Amino Acids                                 │ │
│  │  ◉ Chemical Similarity                                   │ │
│  │                                                          │ │
│  └─────────────────────────────────────────────────────────┘ │
│    ☒ Show SigA Distribution        │ Start Similarity Search │ │
└─────────────────────────────────────────────────────────────┘
```

Beispiel 3.1

Selected Results of Sequence Similarity Search

Query Sequence: MYC$HUMAN
  compared from: 1 to: 439
          with: Protein Database, Release 20
          mode: chemical similarity; positioned mismatches
          date: 31 JUL 92
  Minimum SigA: 28
  Total Matches: 5705
  Matches/Entry: 190.2 ±

| No. | Identifier | Length | SigA | SigB | Short Description | page 1 |
|---|---|---|---|---|---|---|
| 1 | MYC$HUMAN | 439 | 429 | 373 | MYC PROTO-ONCOGENE PROTEIN (GENE NAME: C-MYC) - HOMO ENS | |
| 2 | MYC$PANTR | 439 | 421 | 365 | MYC PROTO-ONCOGENE PROTEIN (GENE NAME: C-MYC) - PAN LODY | |
| 3 | MYC$FELCA | 439 | 341 | 285 | MYC PROTO-ONCOGENE PROTEIN (GENE NAME: C-MYC) - FELIS S | |
| 4 | MYC$FLV | 439 | 339 | 283 | MYC TRANSFORMING PROTEIN (GENE NAME: V-MYC) - FELINE EMI | |
| 5 | MYC$FLVTT | 437 | 331 | 275 | MYC TRANSFORMING PROTEIN (GENE NAME: V-MYC) - FELINE EMI | |
| 6 | MYC$MOUSE | 439 | 328 | 272 | MYC PROTO-ONCOGENE PROTEIN (GENE NAME: C-MYC) - MUS ULUS | |
| 7 | MYC$RAT | 439 | 327 | 271 | MYC PROTO-ONCOGENE PROTEIN (GENE NAME: C-MYC) - RATTUS E | |
| 8 | MYC1$MARMO | 465 | 324 | 268 | TRANSFORMING PROTEIN HCR-MYC - MARMOTA MONAX DCHUCK) | |
| 9 | MYC$CHICK | 416 | 193 | 138 | MYC PROTO-ONCOGENE PROTEIN (GENE NAME: C-MYC) - GALLUS U | |
| 10 | MYC$AVIM2 | 421 | 192 | 137 | MYC TRANSFORMING PROTEIN (GENE NAME: V-MYC) - AVIAN OCYT | |
| 11 | MYC$AVIOK | 416 | 185 | 130 | MYC TRANSFORMING PROTEIN (GENE NAME: V-MYC) - AVIAN OVIR | |
| 12 | MYC$AVIMC | 422 | 176 | 121 | MYC TRANSFORMING PROTEIN (GENE NAME: V-MYC) - AVIAN OCYT | |
| 13 | MYC$AVIMD | 422 | 171 | 116 | MYC TRANSFORMING PROTEIN (GENE NAME: V-MYC) - AVIAN OCYT | |
| 14 | MYC$AVIME | 423 | 153 | 98 | MYC TRANSFORMING PROTEIN (GENE NAME: V-MYC) - AVIAN OVIR | |
| 15 | MYC2$XENLA | 420 | 140 | 85 | MYC II PROTO-ONCOGENE PROTEIN (GENE NAME: C-MYC II) - PU | |
| 16 | MYC1$XENLA | 419 | 139 | 84 | MYC I PROTO-ONCOGENE PROTEIN (GENE NAME: C-MYC I) - PUS | |
| 17 | MYC$SALGA | 414 | 133 | 78 | MYC PROTO-ONCOGENE PROTEIN (GENE NAME: C-MYC) GMENT) - S | |
| 18 | TRX$DROME | 3759 | 126 | 49 | TRITHORAX PROTEIN (GENE NAME: TRX) - DROSOPHILA NOGASTER | |
| 19 | APB$HUMAN | 4563 | 122 | 43 | APOLIPOPROTEIN B-100 PRECURSOR (APO B-100/APO B-48) E NA | |
| 20 | RYNR$HUMAN | 5032 | 122 | 42 | RYANODINE RECEPTOR (GENE NAME: RYR1 OR RYDR) - HOMO ENS | |
| 21 | DMD$HUMAN | 3685 | 114 | 37 | DYSTROPHIN (GENE NAME: DMD) - HOMO SAPIENS (HUMAN) | |
| 22 | RYNR$RABIT | 5037 | 114 | 34 | RYANODINE RECEPTOR - ORYCTOLAGUS CUNICULUS (RABBIT) | |
| 23 | DMD$CHICK | 3660 | 107 | 30 | DYSTROPHIN - GALLUS GALLUS (CHICKEN) | |
| 24 | IRA1$YEAST | 2938 | 101 | 26 | INHIBITORY REGULATOR PROTEIN IRA1 (GENE NAME: IRA1 OR ) | |
| 25 | DYHC$TRIGR | 4466 | 105 | 26 | DYNEIN BETA CHAIN, CILIARY - TRIPNEUSTES GRATILLA AIAN S | |
| 26 | IRA2$YEAST | 3079 | 99 | 24 | INHIBITORY REGULATOR PROTEIN IRA2 (GENE NAME: IRA2) - HA | |
| 27 | SPCA$DROME | 2415 | 92 | 19 | SPECTRIN ALPHA CHAIN (GENE NAME: SPEC-A) - DROSOPHILA NO | |
| 28 | POLG$TVMV | 3005 | 93 | 18 | GENOME POLYPROTEIN (34 KD PROTEIN; HELPER COMPONENT EIN; | |
| 29 | POLG$HCVA | 3898 | 95 | 17 | GENOME POLYPROTEIN - HOG CHOLERA VIRUS (STRAIN ALFORT) N | |
| 30 | POLG$KUNJM | 3433 | 93 | 17 | GENOME POLYPROTEIN (CAPSID PROTEIN C; ENVELOPE OPROTEIN | |

Plot of TRX$DROME vs MYC$HUMAN

```
3200 ┤

2400 ┤

1600 ┤

 800 ┤

      ├────────┼────────┼────────┼────────
          90      180      270      360
```

Current comparison
X-Scale: X-Sequence: MYC$HUMAN(439 aa); Analyzed from: 1 to: 439
Y-Scale: Y-Sequence: TRX$DROME(3759 aa); Analyzed from: 1 to: 3759
Minimal match length: 3; Number of mismatches: 0
100 % compared; 240 matches found; Similarity:   2.49%(3/1)
Sam, 1. Aug 1992   15:39 Uhr



# EP 0 582 755 A1

Beispiel 3.3

Geringe Ähnlichkeit

Plot of MYC C Exon 2 vs. GGCMYCA

Sequence Comparison

X-Axis:
MYC C Exon 2
from: 1   to: 1534

Y-Axis:
GGCMYCA
from: 1   to: 3517

Beispiel 3.4

## Plot of MYC$SALGA vs MYC$HUMAN

Current comparison
X-Scale: X-Sequence: MYC$HUMAN(439 aa); Analyzed from: 1 to: 439
Y-Scale: Y-Sequence: MYC$SALGA(414 aa); Analyzed from: 1 to: 414
Minimal match length: 3; Number of mismatches: 0
100 % compared; 75 matches found; Similarity:  24.14%(3/1)
Sam, 1. Aug 1992   15:41 Uhr

Beispiel 3.5

## Plot of MYC$RAT vs MYC$HUMAN

```
Current comparison
X-Scale: X-Sequence: MYC$HUMAN(439 aa); Analyzed from: 1 to: 439
Y-Scale: Y-Sequence: MYC$RAT(439 aa); Analyzed from: 1 to: 439
Minimal match length: 3; Number of mismatches: 0
100 % compared; 88 matches found; Similarity:  90.94%(3/1)
Sam, 1. Aug 1992   15:21 Uhr
```

**Patentansprüche**

1. Verfahren zum Einordnen von Informationsträgern in Datenbanken für die Durchführung von DNA- und Proteinsequenzvergleichen, bei denen Sequenzen in ihren Eigenschaften miteinander verglichen werden, **dadurch gekennzeichnet,** daß die Sequenz in Strukturwörter zerlegt und ausgebildet wird, wobei den Strukturwörtern Informationen zugeordnet werden, durch welche die Struktur, die Struktur-Funktionsbeziehungen sowie Ähnlichkeitsmerkmale erfaßt und verarbeitbar abgelegt werden.

2. Verfahren nach Anspruch 1, **dadurch gekennzeichnet,** daß die Strukturwörter bestehend aus Nukleotiden für Sequenzvergleiche mit DNA-Sequenzen verwendet werden.

3. Verfahren nach Anspruch 1, **dadurch gekennzeichnet,** daß die Strukturwörter bestehend aus Aminosäuren für den Vergleich mit Proteinsequenzen verwendet werden.

4. Verfahren nach Anspruch 1, **dadurch gekennzeichnet,** daß die Strukturwörter bestehend aus einen Code, der die chemischen und sterischen Eigenschaften der Aminosäuren in Gruppen zusammenfaß für den Vergleich mit Proteinsequenzen verwendet werden.

5. Verfahren nach Anspruch 1 und 2, **dadurch gekennzeichnet,** daß DNA-Strukturwörter solche Ausbildung erhalten, daß in ihnen die Degeneration des genetischen Codes und die DNA-Erkennungseigenschaften sequenzspezifisch bindender Proteine berücksichtigt werden.

6. Verfahren nach einen der vorhergehenden Ansprüche, **dadurch gekennzeichnet,** daß die Proteinstrukturwörter aus zwei Strukturparametern zusammengefügt werden.

7. Verfahren nach Anspruch 6, **dadurch gekennzeichnet,** daß ein Strukturparameter aus der Strukturwörterlänge gebildet wird.

8. Verfahren nach Anspruch 6, **dadurch gekennzeichnet,** daß ein Strukturparameter aus der Gewichtung (SigS) gebildet wird.

9. Verfahren nach Anspruch 2 und 4, **dadurch gekennzeichnet,** daß an definierten Positionen der Strukturwörter Mismatches (N) zugelassen werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | PROCEEDINGS OF THE 1988 INTERNATIONAL CONFERENCE ON PARALLEL PROCESSING 4. Mai 1988, PENNSYLVANIA, US Seiten 257 - 263 E. LANDER ET AL. 'Protein sequence comparison on a data parallel computer' * Zusammenfassung * --- | | G06F15/401 |
| A | COMPUTER JOURNAL Bd. 30, Nr. 5, Oktober 1987, LONDON GB Seiten 420 - 424 A.F.W. COULSON ET AL. 'Protein and nucleic acid sequence database searching: a suitable case for parallel processing' * Introduction * ----- | | |
| | | | **RECHERCHIERTE SACHGEBIETE (Int. Cl.5 )** |
| | | | G06F |

Der vorliegende Recherchenbericht wurde für alle Patentansprüche erstellt

| Recherchenort | Abschlußdatum der Recherche | Prüfer |
|---|---|---|
| DEN HAAG | 24 MAI 1993 | KATERBAU R.E. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.....................................................................................
& : Mitglied der gleichen Patentfamilie, übereinstimmendes Dokument

EPO FORM 1503 03.82 (P0403)